(19) Europäisches Patentamt
European Patent Office
Office européen des brevets

(11) EP 2 027 772 A1

(12) # EUROPÄISCHE PATENTANMELDUNG

(43) Veröffentlichungstag:
**25.02.2009 Patentblatt 2009/09**

(51) Int Cl.:
***A01N 43/16*** *(2006.01)* ***A01N 65/00*** *(2009.01)*
***A01P 17/00*** *(2006.01)*

(21) Anmeldenummer: **08007830.6**

(22) Anmeldetag: **23.04.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **31.05.2007 DE 102007026053**

(71) Anmelder: **Beiersdorf AG**
**20245 Hamburg (DE)**

(72) Erfinder:
- **von der Fecht, Stephanie**
  **22880 Wedel (DE)**
- **Schulz, Jens**
  **25462 Rellingen (DE)**
- **Kröpke, Rainer**
  **22869 Schenefeld (DE)**
- **Nielsen, Jens**
  **24558 Henstedt-Ulzburg (DE)**

(74) Vertreter: **Hartmann, Jost**
**Beiersdorf AG**
**Unnastrasse 48**
**20245 Hamburg (DE)**

(54) **Insektenschutzmittel mit guter Hautverträglichkeit**

(57) Die Erfindung beschreibt tnsektenabwehrende Zubereitungen umfassend einen relativ hohen Anteil an einen oder mehreren Insektenrepellentwirkstoffen gewählt aus Dihydronepetalactonen und/oder Extrakten der Katzenminze und ein oder mehrere Substanzen mit einem log P-Wert im Bereich von - 2,5 bis +2,5.

Die Zubereitungen zeichnen sich trotz des hohen Insektenrepellentgehaltes durch eine gute Hautverträglichkeit aus.

EP 2 027 772 A1

## Beschreibung

Die vorliegende Erfindung betrifft kosmetische Formulierungen zur Insektenabwehr umfassend einen relativ hohen Anteil an Insektenrepellentwirkstoffen gewählt aus Dihydronepetalacton und/oder Extrakten der Katzenminze und ein oder mehrere Substanzen mit einem log P-Wert im Bereich von - 2,5 bis +2,5.
Die Kombination ermöglicht die Bereitstellung eines wirksamen Insektenschutzmittels mit gleichzeitig guter Hautverträglichkeit.

Insektenschutzmittel (Insektenvertreibungsmittel, Insektenabwehrmittel. Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Als Repellent-Wirkstoff ist beispielsweise 1-Piperidincarboxylsäure 2-(2-hydroxyethyl)-1-methylpropylester (INN: Icaridin, CAS-Nummer. 119515-38-7, Elincs-Nummer: 423-210-8) bekannt, der die folgende Struktur aufweist:

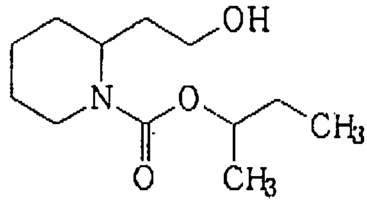

Ein weiterer eingesetzter Repellent-Wirkstoff ist der 3-(N-n-Butyl-N-acetyl-amino)propion-säureethylester (auch als Ethylbutylacetylaminopropionat oder Repellent 3535 bezeichnet), welcher sich durch die folgende Strukturformel auszeichnet:

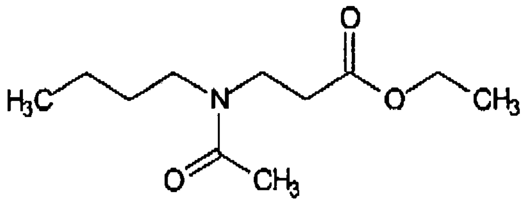

Nicht zuletzt kennt der Fachmann den Repellent-Wirkstoff N,N-Diethyl-3-methylbenzamid (Handelsbezeichnung: Meta-delphene, DEET), der die folgende Struktur aufweist:

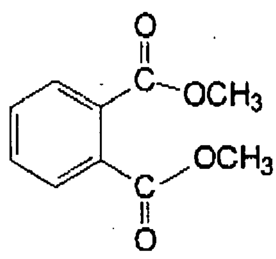

Diese Stoffe sind als Repellentwirkstoffe gegen Insekten, insbesondere Mücken oder Zecken, vielfach beschrieben, wie z.B. in DE 2246433, DE 19645250, DE 19645920.

In der WO 2006096876 und WO 2005034626 werden Dihydronepetalactone als Repellentien gegen Insekten offenbart. Der Offenbarungsgehalt zu Dihydronepetalacton der WO 2006096876 und WO 2005034626 sind hiermit explizit als erfindungsgemäß mitumfasst.

Katzenminze oder auch Nepeta ist als Mückenabwehrmittel bekannt und soll eine etwa zehn Mal stärkere Abwehr gegen Mücken als herkömmliche Abwehrmittel aufweisen.

In der DE 102005033845 werden des weiteren u.a bestimmte Parfuminhaltsstoffe zur Abwehr von Mücken, Sandfliegen, Läusen, Bremsen, Wespen, Bienen, Fliegen und Zecken erwähnt.

Ein Problem ist dabei, dass ein Repellentwirkstoff, der beispielweise gegen Mücken abwehrend wirkt, diese Abwehrwirkung nicht oder nur eingeschränkt gegen andere Insekten aufweist.

Um wirksam Insektenabwehrend zu wirken müssen die Repellentien zu einem relativ hohen Anteil zugesetzt werden. Um einen Vollschutz gegen Zecken oder auch der sehr aggressiven Anopheles Mücke, zu erzielen, muss ein Zusatz von bis 20% des Repellents zugesetzt werden. Problem dabei ist, dass Zubereitungen mit einer hohen Konzentration an Repellentien bei Menschen mit besonders empfindlicher Haut in Einzelfällen zu Hautirritationen führen können. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Die Zubereitungen des Standes der Technik haben bei derartig hohen Einsatzkonzentrationen den Nachteil, insbesondere bei Menschen mit empfindlicher oder zu Allergien neigender Haut nicht besonders verträglich zu sein und in Einzelfällen zu Hautreizungen zu führen.

Wünschenswert ist es eine Zubereitung zur Verfügung zu stellen, die einerseits insektenabwehrend wirkt und andererseits hautverträglich gestaltet ist und vorteilhaft einen für die menschliche Nase angenehm wohlriechenden Duft verströmt.

Es war daher die Aufgabe der vorliegenden Erfindung, kosmetische Zubereitungen mit einem Gehalt an Dihydronepetalactone zu entwickeln, die besonders hautfreundlich und hautverträglich sind, und ein reduziertes Hautreizungspotential aufweisen.

Die Erfindung beschreibt eine Zubereitung umfassend einen oder mehrere Insektenrepellentwirkstoffe gewählt aus Dihydronepetalactonen und/oder Extrakten der Katzenminze und ein oder mehrere Substanzen mit einem log P-Wert im Bereich von - 2,5 bis +2,5. Insbesondere ist der Anteil an Repellentien in der Zubereitung relativ hoch, d.h. bis zu 40 Gew.% und bevorzugt im Bereich von 5 bis 20 Gew.%, bezogen auf die Gesamtmasse der Zubereitung.

Dihydronepetalactone sind beispielweise in der WO 2006096876 und WO 2005034626 beschrieben, die umfänglich zum Offenbarungsgehalt der vorliegenden Erfindung zählen.

Die Katzenminzen (Nepeta) bezeichnen eine Pflanzengattung der Familie der Lippenblütler (Lamiaceae). Erfindungsgemäß werden als Extrakte alle möglichen Extrakte, Lösungen oder Dispersionen der Nepetapflanze oder deren Teile verstanden.

Bei den in den Nepeta enthaltenen ätherischen Ölen handelt es sich in den Hauptbestandteilen um Citral, Citronellol, Geraniol und Limonen, sowie Nepetalacton, Gerbund Bitterstoffe. Der Wirkstoff, der die Pflanze für Katzen so unwiderstehlich macht, ist Actinidin, ein iridoides Glykosid, das dem vergleichbaren Wirkstoff des Baldrian sehr ähnlich ist (BOWN, 1995). Die Nepeta enthält etwa 0,2-0,7 % ätherische Öle.

Der ölige Extrakt der Katzenminze, insbesondere aufgereinigt, ist wie jedes andere Naturöl sehr schlecht spreitend und sehr polar. Es ist deshalb unkosmetisch und verleiht den herkömmlichen kosmetischen Formeln eine ölig, fettige Phase. Es wird als schwer verteilbar wahrgenommen. Der polare Charakter des Extraktes führt zudem sehr schnell zu Instabilitäten, wie z.B. Öl- oder Wasserabscheidung.

Erfindungsgemäß ist diese Schwierigkeit der kosmetischen Formulierung durch die Kombination mit Substanzen mit einem log P im Bereich von -2,5 bis +2,5 überwunden worden.

Die erfindungsgemäße Zubereitung kann die Dihydronepetalactone und/oder Extrakte der Katzenminze in unterschiedlichen Formen enthalten. Bevorzugt enthält die Zubereitung eine aufgereinigte Repellentienmischung, die zu etwa 90 Gew.% Dihydronepetalacton und 2 Gew.% Dehydronepetalacton, bezogen auf die Mischungsmasse, als wirksame Repellentien umfasst.

Es ist erfindungsgemäß vorteilhaft, wenn die Repellentien in einer Gesamtkonzentration von 0,1 bis 40 Gew. % und erfindungsgemäß bevorzugt, wenn die Repellentien in einer Gesamtkonzentration von 5 bis 20 Gew.%, jeweils bezogen auf das Gesamtgewicht der Zubereitung, in dieser enthalten sind.

Bevorzugt ist eine erfindungsgemäße Zubereitung, die weiteren Insektenrepellentien, wie z.B. 1-Piperidincarboxylsäure 2-(2-hydroxyethyl)-1-methylpropylester (INN: Icaridin) und/oder N,N-Diethyl-3-Methylbenzamid (DEET) und/oder Ethylbutylacetylaminopropionat (EBAAP) umfasst.

Ob eine Substanz einen log P-Wert von -2,5 bis +2,5 aufweist und demnach erfindungsgemäß ist, lässt sich mit der folgenden Messmethode bestimmen:

Der Verteilungskoeffizient Log P ist der Quotient der Konzentrationen einer Substanz in der Wasserphase bzw. der Octanolphase nach Einstellung des Verteilungsgleichgewichtes. Die analytische Bestimmung der Konzentrationen wird meist UV-spektroskopisch durchgeführt.

Der Octanol/Wasser-Verteilungskoeffizient (P) einer Substanz "A" ist definiert als (Gleichung 1):

$$P = \frac{\text{Konzentration—Lipidphase}}{\text{Konzentration – Wasserphase}} \Rightarrow \log P = \log \frac{[\text{Octanol}]}{[\text{Wasser}]} \qquad \text{(Gleichung 1)}$$

Log P > 0 => Substanz löst sich besser in Octanol
Log P <0 => Substanz löst sich besser in Wasser

Es können auch direkt die Extinktionen der UV-spektroskopischen Konzentrationsbestimmung in die Gleichung eingesetzt werden. Bei einem Volumenverhältnis Octanol/Wasser 1/1 gilt (Gleichung2):

$$\text{Log P=log E0-E1} \qquad \text{(Gleichung 2)}$$

E0 = Extinktion Maßlösung; E1 = Extinktion der Probe in Wasser ( Maßlösung in Wasser) Versuchsbeschreibung:

Geräte: Maßkolben für Standardlösung (20-100ml), DC-Kammer (klein), DC-Platten-Alugram, 5 Schütteltrichter, Klemme, Stativring, Zentrifuge, Zentrifugengläser, UV-Spectrometer, Quarzküvetten (1 cm).
Chemikalien: Octanol, Wasser dest., Substanz

Die erhaltene Probe wird mittels Dünnschichtchromatographie auf ihre Reinheit untersucht, da schon geringe Verunreinigungen eine starke Auswirkung auf die Extinktion und damit auf den mit Hilfe der Extinktion ermittelten Log P-Wert haben.
Es wird danach eine Standardlösung (in der Regel 10-4 M) in dem jeweiligen Puffer hergestellt (Maßkolben), die im Ausschüttelexperiment als auch als Standard für die UV-Messung benutzt wird
Es wird ein UV-Spektrum der Standardlösung aufgenommen (200nm - 300nm Scanmodus).
Die Standardlösung sollte bei der Messung im Maximum eine Extinktion zwischen 0,25 und 1,0 aufweisen. Ist dies nicht der Fall, ist entsprechend zu verdünnen.
15 ml der Maßlösung werden mit 15 ml des entsprechenden, fehlenden Lösungsmittels in einen Schütteltrichter gefüllt (Volumenverhältnis organische Phase / wässrige Phase, 1/1) und 15-20 min. gleichmäßig geschüttelt. Zur Komplettierung der Phasentrennung wird 20 min bei 2500-3000 rpm zentrifugiert.
Alternativ kann das Schütteln der Probe in 2ml-Eppendorfcaps erfolgen. Hierzu werden 1 ml der Maßlösung und 1ml des fehlenden Lösungsmittels 15 - 20 min. in einem "2ml-Eppi' in einem speziellen Schüttler durchmischt, vervollständigt wird die Trennung der beiden Phasen wiederum durch Zentrifugation.
Ein dritte Möglichkeit - und auch die Methode der Wahl - bietet eine spezielle Schüttelapparatur. Hier können mit je 2 (3) ml Octanol und Wasser (bzw. Puffer) befüllte verschließbare Zentrifugenröhrchen eingespannt und für mindestens 20 min. geschüttelt werden. Danach schließt sich wieder der oben beschriebene Zentrifugationschritt an.
Während der Zentrifugation wird die Maßlösung noch einmal gegen das entsprechende Lösungsmittel UV-spektroskopisch vermessen (Wert = E0). Nach Beendigung der Zentrifugation werden gleiche Mengen (Volumen) aus der Wasser bzw. Octanolphase entnommen und gegen das entsprechende Lösemittel UV-spektroskopisch vermessen, Die erhaltenen Extinktionen können direkt zur Bestimmung des Verteilungskoeffizienten benutzt werden ( s. Gleichung (2)).
Darüber hinaus kann der log P-Wert nach der Methode von "Hansch und Leo", die 1979 veröffentlicht worden ist, berechnet werden.
Die Hansch und Leo Methode beinhaltet hergeleitete Fragmentkonstanten f (Atome oder Atomgruppen) und Strukturfaktoren F (berücksichtigen die räumliche Anordnung des Moleküls) Weiterführende Literatur findet man in

- Seydel, Schaper, Chemische Struktur und biologische Aktivität von Wirkstoffen, 1979, Verlag: Chemie Weinheim
- Strategy of Drug Design: A guide to biological Activity, Purcell, Bass, Clayton, 1973, J. Wiley and Sons
- Rekker, Mannhold, Calculation of Drug Lipophilicity, 1992, Verlag Chemie Weinheim
- Kubiny, H.; QSAR: Hansch Analyses and Related Approaches, Vol 1, Kap. 4.5, 5. 77 ff Brehm, O., Ohlmeyer, I. and Fels, G.; Automatisierte pKa- und logP-Bestimmung. GIT Labor-Fachzeitschrift, 1997, 41, 368-374.

Es ist erfindungsgemäß vorteilhaft, wenn als Substanzen mit einem log P-Wert von -2,5 bis +2,5 eine oder mehrere Verbindungen gewählt werden aus der Gruppe der mehrwertigen Alkohole, linearen Alkohole, verzweigten

Alkohole, C5-C10 Alkylalkohole, Polyglycerine und/oder hydrophile Wirkstoffe.

Es ist erfindungsgemäß bevorzugt, wenn als Substanzen mit einem log P-Wert von -2,5 bis +2,5 eine oder mehrere Verbindungen gewählt aus der Gruppe C5-C10 Alkylalkohole, verzweigten Alkohole sowie hydrophile Wirkstoffe eingesetzt werden.

Erfindungsgemäß besonders bevorzugt ist der Einsatz von C5-C10 Alkylalkohole und hydrophilen Wirkstoffen als Substanzen mit einem log P-Wert von -2,5 bis +2,5.

Beispiele besonders geeigneter Substanzen mit einem log P-Wert von -2,5 bis + 2,5 sind die Substanzen in der nachstehenden Tabelle:

| Name | CAS-Nr.: | Log P-Wert |
|---|---|---|
| 2,2 Bishydroxymethyl 1,3 Propanediol | 115-77-5 | -2,4 |
| 1,2,3, Propantriol | 56-81-5 | -2,3 |
| 1,2 Propandiol | 57-55-6 | -1,3 |
| Butane-1,4diol, Tetramethylene Glucol | 110-63-4 | -1 |
| 2-Ethyl-2-Hydroxymethyl 1,3 Propandiol | 77-69-6 | -0,8 |
| 1,3 Butandiol | 107-88-0 | -0,7 |
| 2-Methyl-1,3-Propanediol | 2163-42-0 | -0,7 |
| 1,5 Pentandiol | 111-29-5 | -0,6 |
| 1,3-Dimethoxy-2-propanol | 623-69-8 | -0,5 |
| 1,2 Pentandiol | 5343-92-0 | -0,3 |
| Neopentanediol, Neopentyleme Glyol | 126-30-7 | -0,3 |
| 3-Methyl 1,3 Butanediol | 2568-33-4 | -0,3 |
| 3,3,7,7 Tetrahydroxymethyl 5 oxanonan | 23235-61-2 | -0,3 |
| 1,6 Hexandiol | 629-11-8 | -0,1 |
| 2-Methyl-2,4 Pentandiol | 107-41-5 | 0 |
| 1-Butanoylglycerol, 1-Glyceryl-monobutyrate | 557-25-5 | 0 |
| 1,2,3 Octanetriol | 112196-85-7 | 0,15 |
| 1,2 Hexandiol | 6920-22-5 | 1 |
| 1,2 Octandiol | 1117-86-8 | 1,3 |
| 1,2 Heptanediol -- | 3710-31-4 | 1,5 |
| 2-Butyl-2-ethyl)-1,3-propanediol | 115-84-4 | 1,8 |
| 3-[(2-Ethylhexyl)oxy]-1,2 propandiol | 70445-33-9 | 2,1 |
| 1,2-Decandiol | 1119-86-4 | 2,4 |
| 1,2 Nonanediol | 42789-13-9 | 2,5 |

Der n-Oktanol-Wasser-Verteilungskoeffizient $K_{ow}$ oder der dekadische Logarithmus des Verteilungskoeffizient wird auch als log P bezeichnet ist ein dimensionsloser Verteilungskoeffizient, der das Verhältnis der Konzentrationen einer Chemikalie in einem Zweiphasensystem aus n-Octanol und Wasser angibt.

Der Log P-Wert ist somit ein Modellmaß für die Polarität bzw. Wasser-/Fettlöslichkeit der Chemikalie: Je höher der Koeffizient, desto stärker die Tendenz des Stoffes, sich z.B. im Fettgewebe von Organismen anzureichern (Depot-Effekt). Die Substanzen mit einem log P-Wert von -2,5 bis + 2,5 haben keinen negativen Einfluss auf die Hautverträglichkeit.

Neben der Insektenabwehr sind aber vor allem die Hautverträglichkeit und das Hautgefühl entscheidende Eigenschaften eines topisch zu applizierenden insbesondere kosmetischen Produktes.

Es nützt eine hervorragende Repellentwirksamkeit nichts, wenn das Kosmetikprodukt zu Unverträglichkeiten führt.

Eine der häufig auftretenden Hautunverträglichkeiten ist der sog. Stinging-Effekt, der sich insbesondere im Gesicht unangenehm bemerkbar macht. Der Stinging-Effekt tritt insbesondere im Nasolabial-Bereich auf und bedeutet ein subjektives Missempfinden der Probanden.
Um solche unerwünschten Effekte für Kosmetika möglichst auszuschliessen werden vor Produkteinführung sog. Stinging-Tests durchgeführt. Jeweils zwei Produkte werden dabei im dirketen Paarvergleich auf ihre Hautverträglichkeit getestet. Als interner High-Standard (Benchmark) wird dabei ein als hautverträglich bekanntes handelsübliches Produkt verglichen (NIVEA Visage Day Cream normal mixed).

In zahlreichen Untersuchungen wurde nun festgestellt, dass ein wesentlicher Beitrag zum Stinging durch die in vielen Kosmetika enthaltenen Parabene hervorgerufen wird, insbesondere wenn diese mit einem Anteil von mehr als 0,3 Gew.% in der Rezeptur vorliegen.
Parabene werden als Konservierungsmittel eingesetzt, so dass deren Ersatz mitunter schwierig ist.

Es wurde nun überaschenderweise fest gestellt, dass die erfindungsgemäßen Zubereitungen sehr gute Repellentwirksamkeiten zeigen aber einen Stinging-Effekt nicht aufweisen.
Die erfindungsgemäßen Zubereitungen lassen sich daher bevorzugt mit einem Anteil von bis zu 0,3 Gew.% Konservierungsmittel, wie insbesondere Parabene, herstellen.

Bevorzugt kann auf den Zusatz von Konservierungsmitteln, wie z.B. Parabenen, ganz verzichtet werden.
D.h es ist erstmals möglich Repellenthaltige Zubereitungen zur Verfügung zu stellen, die gleichzeitig verbesserte Verträglichkeit aufweisen und insbesondere keine Stinging-Effekte aufweisen.
Die erfindungsgemäßen Zubereitungen sind daher bevorzugt frei von bakteriziden-, fungiziden-, sporicidally d.h. sporenabtötenden, wirksamen Mitteln und/oder frei von Konservierungsmitteln. Weiterhin sind sie bevorzugt frei von Formaldehyd, Formaldehyddonatoren wie Diazolidinyl urea, Imidazolidinyl urea und/oder DMDM Hydantoin sowie insbesondere frei von Benzoesäure, p-Hydroxybenzoesäure und/oder oder deren Derivate oder Salze. Bevorzugt umfassen die erfindungsgemäßen Zubereitungen weniger als 0,3 Gew.% bzw. insbesondere sind sie frei von Parabenen, insbesondere frei von Methylparaben, Ethylparaben, Propylparaben, Isopropylparaben, Butylparaben, Isobutylparaben und/oder Benzylparaben. Auch 2,4-Dichlorobenzylalkohol, 4-Chloro-3,5-dimethyl-phenol, 2-Dromo-2-nitropropane-1,3-diol und/oder Iodopropinylbutylearbamate (IPBC) sowie Quaternium-15 (CAS 51229-78-8), Methyl-chloroisothiazolinone und/oder Methylisothiazolinon sind in den erfindungsgemäßen Zubereitungen nicht bzw. zu weniger als 0,3 Gew.% zu finden.
Die erfindungsgemäßen Zubereitungen sind besonders bevorzugt Parabenfrei.
Es bedeutet erfindungsgemäß "frei von" ein Anteil von weniger als 0,1 Gew.%, bezogen auf die Gesamtmasse der Zubereitung. Bevorzugt beträgt der Anteil an den zuvor genannten Mitteln und insbesondere Parabenen 0 Gew.%.

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil an Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repelient-Wirkstoffen einzusetzen.

Die Repellent-Wirkstoffe haben einen auch für die menschliche Nase wahrnehmbaren und auf Mitmenschen nicht gerade anziehend wirkenden Eigengeruch, der die Anwendung von Insektenabwehrmitteln nicht gerade attraktiv macht.

Vorteilhaft werden der erfindungsgemäßen Zubereitung ein oder mehrere Parfuminhalststoffe zugesetzt, gewählt aus der Gruppe

- 1,3,4,6,7,8-Hexahydro-4,6,6,7,8,8-hexamethylcyclopenta-gamma-2-benzopyran
- 2,6-Dimethyl-7-octen-2-ol
- 2-Acetonapthone-1,2,3,4,5,6,7,8-octahydro-2,3,8,8-tetramethyl
- 2-Isobutyl-4-hydroxy-4-methyltetrahydropyran
- 2-tert-Pentylcyclohexylacetat
- 3,7-Dimethyl-2,6-octadien-1-ol
- 3-Methyl-5-phenyl-1-pentanol
- 7-Acetyl-1,1,3,4,4,6-hexamethyltetralin
- Adipinsäurediester
- alpha-Amylcinnamaldehyd
- alpha-Isomethylionon
- Alpha-Methylionon
- Amyl C Butylphenylmethylpropionalcinnamal
- Amylcinnamylalkohol

- Amylsalicylat
- Anisalkohol
- Benzoin
- Benzylacetat
- Benzylalkohol
- Benzylbenzoat
- Benzycinnamat
- Benzylsalicylat
- Bergamotöl
- bitteres Orangenöl
- Butylphenylmethylpropioal
- Cardamomöl
- Cedrol
- Cinnamal
- Cinnamylalkohol
- Citral
- Citronellol
- Citronellylmethylcrotonat
- Citronenöl
- Coumarin
- Diethylsuccinat
- d-Limonene
- Ethylenbrassylate
- Ethyllinalool
- Eugenol
- Evernia Furfuracea Extract
- Evernia Prunastri Extract
- Famesol
- Geraniol
- Guajakholzöl
- Heliotropin
- Hexylcinnamal
- Hexylsalirylat
- Hydroxycitronellal
- Hydroxyisohexyl 3-Cyclohexencarboxaldehyde
- Isoeugenol
- Lavendelöl
- Lemonenöl
- Limonen
- Linalool
- Linalylacetat
- Mandarinenöl
- Menthyl PCA
- Methyl-2 Octynoat
- Methylbenzoat
- Methylcedrylketone
- Methyldihydrojasmonate
- Methylheptenon
- Muskatnussöl
- p-t-Butyl-alpha-methyldihydrocinnamic aldehyde
- Rosmarinöl
- süßes Orangenöl
- Terpineol
- Tonkabohnenöl
- Triethylcitrat und/oder
- Vanillin.

Vorteilhaft ist der Zusatz von Hexylsalicylat, Linalylacetat und/oder 2-Isobutyl-4-hydroxy-4-methyltetrahydropyran, die den Eigengeruch des Dihydronepetalactons besonders gut maskieren ohne einen anlockenden Effekt auf die Insekten auszuüben.

Die erfindungsgemäßen Parfuminhaltstoffe, ein oder mehrere, sind bevorzugt zu einer Gesamtkonzentration von 1 ppm bis 2,5 Gew.%, jeweils bezogen auf das Gesamtgewicht der Zubereitung, in dieser enthalten.

Eine vorteilhafte Ausführungsform im Sinne der vorliegenden Erfindung ist eine kosmetische Zubereitung als Hydrodispersionsgel enthaltend neben den log P-Wert spezifischen Substanzen 15 Gew.% Dihydronepetalacton und 0,004 Gew.% Hexylsalicylat, jeweils bezogen auf das Gesamtgewicht der Zubereitung.

Die Zubereitung ist bevorzugt eine kosmetische Zubereitung und zum Auftragen auf die Haut geeignet.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/S, S/W oder multiple Emulsion, Makro- oder Mikroemulsion), einer Dispersion, einer Pickering-Emulsion, eines Gels, eines Hydrodispersionsgels oder einer wasserfreien Zubereitung vorliegt. Dabei ist es erfindungsgemäß bevorzugt, wenn die kosmetische Zubereitung in Form eines Hydrodispersionsgels vorliegt.

Die Zubereitung kann erfindungsgemäß auch in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Zubereitungen mit Dihydronepetalacton als Wirkstoff können in Form von Lösungen, Emulsionen und Dispersionen hergestellt werden, bei denen der lipophile Wirkstoff von einer wässrigen Phase umgeben ist (z.B. O/W-Emulsion). Durch die Umhüllung des Wirkstoffs mit der wässrigen Phase wird dessen Freisetzung aber gebremst und die Zubereitung selbst ist bei der Anwendung auf der Haut länger wirksam gegen Mücken, Insekten, etc.

Nachteilig am Stande der Technik ist jedoch der Sachverhalt, das diese Zubereitungen aufgrund ihrer wässrigen äußeren Phase nicht besonders wasserfest sind und leicht von der Haut abgespült werden können. Ferner lassen sich in diesen Zubereitungen keine oxidationsempfindlichen, wasserlöslichen Zusatzstoffe einarbeiten.

Diese Mängel lassen sich erfindungsgemäß durch Emulsionen mit einer wässrigen inneren Phase und einer lipophilen äußeren Phase, enthaltend Dihydronepetalactone, beheben.

Besonders vorteilhaft liegen die erfindungsgemäßen Zubereitung daher in Form einer Wlaoder W/S-Emulsion vor.

Die erfindungsgemäßen Zubereitungen weisen dadurch eine verlängerte Wirkdauer und eine erhöhte Wasserfestigkeit auf der Haut auf.

Überraschend war insbesondere der Umstand, dass, obwohl der Repellent-Wirkstoff in der äußeren, lipophilen Phase angereichert ist, bei der Anwendung auf der Haut gleichmäßig über einen Zeitraum von mehreren Stunden an die Umwelt abgegeben wird und somit eine lang anhaltende Repellent-Wirkung entfaltet.

Überraschend war nicht zuletzt, dass bei den erfindungsgemäßen Zubereitungen die Klebrigkeit der Zubereitung auf der Haut im Vergleich zu Produkten des Stand der Technik (mit gleichem Repellent-Gehalt) deutlich reduziert ist.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammameiis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht

der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Als insbesondere kosmetische Zubereitung ist die erfindungsgemäße Zubereitung als topisch anzuwendendes Mittel vorteilhaft geeignet.

Ebenso ist die Verwendung einer erfindungsgemäßen Zubereitung als Insektenabwehrmittel in einer Verdunstungsvorrichtung möglich und bevorzugt.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**W/O-Emulsionen**

| | **1** | **2** | **3** | **4** | **5** |
|---|---|---|---|---|---|
| Triglycerindiisostearat | 1,0 | 0,5 | 0,25 | 2,0 | -- |
| Diglycerindipolyhydroxystearat | 1,0 | 1,5 | 1,75 | 3,0 | -- |
| Paraffinöl | 12,5 | 10,0 | 8.0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Dihydronepetalacton und Katzenminzeextrakt | 5,0 | 10,0 | 7,5 | 13,0 | 7,5 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | -- | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s. | q,s, | q,s, | q,s, |
| Ethanol | 2,0 | --- | 5,0 | --- | --- |
| Capryl-/Capnnsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Wollwachsalkohol | 1,5 | 0,3 | 0,5 | 1,0 | 5,0 |
| Wasser | ad 100 | ad 100 | ad 100 | ad 100 | ad 100 |

**W/O-Emulsionen**

| | **6** | **7** | **8** | **9** | **10** |
|---|---|---|---|---|---|
| PEG-30 Dipolyhydroxystearat | -- | 0,5 | 0,25 | -- | 3,0 |

(fortgesetzt)

| | 6 | 7 | 8 | 9 | 10 |
|---|---|---|---|---|---|
| Wollwachsalkohol | 1,0 | 1,5 | 1,75 | 3,0 | -- |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosplyceride | 2,0 | 1,0 | 2,5 | 5.0 | 0,25 |
| Dihydronepetalacton | 5,0 | 7,5 | 10,0 | 15,0 | 7,5 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Tafelparaffin | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | -- | 5,0 | -- | -- |
| Capryl-/Caprinsäuretriglycerid | 2,0 | 2,5 | 3,0 | 5,0 | 0,5 |
| Natriumdehydracet | --- | -- | 0,05 | -- | -- |
| Kaliumsorbat | 0,3 | 0,4 | 0,25 | 0,15 | -- |
| Talkum | --- | -- | 0,05 | -- | 0,1 |
| Wasser | ad 100 | ad 100 | ad 100 | ad 100 | ad 100 |

**W/S-Emulsion**

| | 11 | 12 | 13 | 14 | 15 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Vaseline | 12,5 | 15 | 28,0 | 25,0 | 17,5 |
| Dimethicon | 5,0 | 13,0 | 5,0 | 12,0 | 15,0 |
| Dihydronepetalacton | 5,0 | 7,5 | 10,0 | 15,0 | 7,5 |
| Mineralöl | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |

(fortgesetzt)

| | 11 | 12 | 13 | 14 | 15 |
|---|---|---|---|---|---|
| Wollwachs | 1,0 | 0,5 | 2,5 | 0,25 | 5,5 |
| Cetyldimethicon | 0,5 | --- | 0,7 | --- | --- |
| Benzylalkohol | --- | -- | 0,05 | -- | 0,1 |
| Wasser | ad 100 | ad 100 | ad 100 | ad 100 | ad 100 |

**W/S-Emulsionen**

| | 16 | 17 | 18 | 19 | 20 |
|---|---|---|---|---|---|
| Cetyl PEG/PPG-10/1 Dimethicone | 1,0 | -- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dihydronepetalacton | 5,0 | 13,0 | 5,0 | 12,0 | 15,0 |
| Mikrowachs | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | -- | -- |
| Natriumlactat | 0,2 | 1,0 | 0.05 | -- | --- |
| Parfum | q,s, | q,s, | q,s, | q,s, | q,s, |
| Wollwachsalkohol | 1,0 | 1,5 | 1,5 | 0,25 | 0,1 |
| Stearyldimethicon | 0,5 | -- | 0,7 | --- | -- |
| Dehydracetsäure | -- | -- | 0,05 | -- | 0,1 |
| modifizierte Stärke | -- | 2,5 | --- | 0,15 | -- |
| Wasser | ad 100 | ad 100 | ad 100 | ad 100 | ad 100 |

**W/O-Emulsionen-parabenfrei**

| | 21 | 22 | 23 | 24 | 25 |
|---|---|---|---|---|---|
| PEG-22 Dodecyl Glycol Copolymer | 5,0 | 1,5 | 0,25 | -- | 3,0 |
| PEG-45 Dodecyl Glycol Polymer | 1,0 | 1,5 | 1,75 | 3,0 | 8,0 |
| Paraffinöl | 12,5 | 10,0 | 8,0 | 5,0 | 7,5 |
| Dihydronepetalacton | 8,0 | 6,0 | 5,0 | 12,0 | 17,5 |
| Tafelparaffin | 2,0 | 1,0 | 2,5 | 5,0 | 0,25 |
| Nachtkerzenöl | 0,5 | 0,75 | 1,0 | 2,0 | 0,25 |
| Vaseline | 0.5 | 1,0 | 0,75 | 3,0 | 0,25 |

(fortgesetzt)

| | 21 | 22 | 23 | 24 | 25 |
|---|---|---|---|---|---|
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Wollwachsalkohol | 0,5 | 1,0 | 7,5 | 0,25 | 2,5 |
| Magnesiumsulfat | 0,5 | 0,6 | 0,5 | 0,7 | 1,0 |
| Glycerin | 3,0 | 5,0 | 10,0 | 15.0 | 1,5 |
| Natriumcitrat | 0,2 | 0,1 | -- | -- | -- |
| Zitronensäure | 0,2 | 0,1 | -- | -- | -- |
| Parfum | q.s. | q.s. | q.s. | q.s. | q.s. |
| 1,3 Butylenglykol | 2,0 | -- | 5,0 | -- | -- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | -- | -- | 0,05 | -- | 0,1 |
| Wasser | ad 100 | ad 100 | ad 100 | ad 100 | ad 100 |

**W/O-Emulsionen**

| | 26 | 27 | 28 | 29 | 30 |
|---|---|---|---|---|---|
| Polyglyceryl-2-dipolyhydroxystearat | 3,0 | --- | 0,25 | -- | 3,0 |
| Polyglyceryl-3 diisostearate | 1,0 | 3,5 | 1,75 | 2,5 | -- |
| PEG-40 sorbitanisostearate | -- | 2,5 | 0,5 | 3,5 | 3,0 |
| Katzenminzeextrakt | 12,5 | 10,0 | 8,0 | 5,0 | 17,5 |
| Mikrowachs | 2,5 | 1,5 | 5,0 | 1,2 | 0,25 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Wollwachsalkohol | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0,8 |
| Parfum | q.s. | q.s. | q.s. | q.s. | q.s. |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

**Silikon in Wasser-Emulsion-parabenfrei**

| | **31** | **32** | **33** | **34** | **35** |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dihydronepetalacton | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Xanthan Gum | -- | 0,1 | -- | 0,25 | 1,0 |
| Panthenol | 0,5 | 7,0 | 0,75 | 0,25 | 0,1 |
| Parfum | q,s, | q,s, | q,s, | q,s, | q,s, |
| Methylisothiazolinone | 0,06 | 0,08 | 0,1 | 0.04 | 0,1 |
| Wollwachsalkohol | 0,5 | 1,5 | 1,0 | 0,75 | 0,25 |
| Wasser | ad 100 | ad 100 | ad 100 | ad 100 | ad 100 |

**O/W-Emulsion-parabenfrei**

| | **36** | **37** | **38** | **39** | **40** |
|---|---|---|---|---|---|
| Glycerylsterat | 1,0 | -- | -- | 0,5 | 0,25 |
| Polyethylenglycol(40)stearat | 10,0 | -- | 5 | -- | -- |
| Triglycerinmethylglucosedistearat | -- | 5,5 | -- | -- | 2,5 |
| Sorbitanstearat | -- | 1,5 | 3 | -- | -- |
| Dihydronepetalacton | 2,5 | 35 | 8,0 | 5,0 | 7,5 |
| Dimethicon | 5,0 | 3,0 | 5,0 | 2,0 | 5,0 |
| Behenylalkohol | 1 | -- | 2 | 1 | -- |
| Stearylalkohol | -- | 1 | -- | 1 | -- |
| Wollwachsalkohol | 5 | -- | 3 | 2 | 2,5 |
| Xanthan Gummi | -- | 1,5 | 0,5 | 0,3 | -- |
| Cetylstearylalkohol | -- | 5 | 1 | 1 | -- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q.s. | q.s. | q.s. | q.s. | q.s. |
| Methylisothiazolinone | 0,1 | 0,04 | 0,06 | 0,08 | 0,1 |
| Glycerin | 5 | 10 | 3 | 15 | 7,5 |
| modifizierte Stärke | -- | 2,5 | -- | 0,15 | -- |
| Wasser | ad 100 | ad 100 | ad 100 | ad 100 | ad 100 |

**O/W-Emulsion**

| | **41** | **42** | **43** | **44** | **45** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | -- | 8 | -- | -- | -- |
| Dihydronepetalacton | 5,0 | 7,5 | 12,5 | 10,0 | 20,0 |
| Stearylalkohol | 3 | 2 | -- | 2 | -- |
| Cetylstearylalkohol | 3 | 4 | -- | -- | 2 |
| Mineralöl, dickflüssig | 0,5 | 0,75 | 1,0 | 2,0 | 0,25 |
| Vaseline | 5 | 3,5 | 2,5 | 1,0 | 7,5 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | -- |
| Methylisothiazolinone | -- | -- | 0,05 | -- | 0,1 |
| modifizierte Stärke | 0,5 | -- | -- | 0,15 | -- |
| Wasser | ad 100 | ad 100 | ad 100 | ad 100 | ad 100 |

**O/W-Emulsion-parabenfrei**

| | **46** | **47** | **48** | **49** | **50** |
|---|---|---|---|---|---|
| Glycerylsteratcitrat | 1,0 | 5,5 | 0,1 | 0,5 | 0,3 |
| Polyethylenglycol(20)cetearylether | 10,0 | 3,5 | 5 | -- | -- |
| Triglycerinmethylglucosedistearat | -- | 2,5 | -- | -- | 2,5 |
| Dihydronepetalacton + Katzenminzeextrakt | 5 | 35 | 15 | 20 | 2,5 |
| Mikrowachs | 1,0 | -- | 0,5 | 0,75 | 2,25 |
| Dimethicon | 0,5 | 0,5 | 0,75 | 1,5 | 0,2 |
| Behenylalkohol | 1 | -- | 2 | 1 | 0,2 |
| Dicaprylylcarbonat | 3 | 5 | 10 | 15 | 5 |
| Stearylalkohol | -- | -- | -- | 1 | 0,2 |
| Cetylstearylalkohol | -- | -- | 1 | 1 | 0,2 |
| Tocopherol | 0,5 | 0,5 | 0.75 | 0,25 | 0,1 |
| Octyldodecanol | 0,5 | -- | 0,75 | 3,0 | 0,25 |
| Panthenol | 0,5 | -- | 0,75 | 0,25 | 0,1 |
| Carbomer | 0,05 | 1,0 | 0,15 | 0,1 | -- |

(fortgesetzt)

| | 46 | 47 | 48 | 49 | 50 |
|---|---|---|---|---|---|
| Parfum | q,s, | q,s, | q,s, | q,s, | q,s, |
| Caprylic/Capric Triglycerid | 1 | 5 | 3 | 5 | 10 |
| Methylisothiazolinone | 0,04 | 0,08 | 0,1 | 0,1 | 0,06 |
| Phenoxyethanol | -- | 0,5 | -- | 0,15 | -- |
| Sorbitol | 10 | -- | -- | 5 | -- |
| Butylenglykol | -- | -- | -- | 5 | 10 |
| Propylenglykol | -- | -- | 10 | 5 | -- |
| Glycerin | -- | 7,5 | -- | -- | -- |
| Wasser | ad 100 | ad 100 | ad 100 | ad 100 | ad 100 |

**kationische O/W-Emulsionen**

| Beispiel Nummer | 51 | 52 | 53 | 54 | 55 |
|---|---|---|---|---|---|
| Distearyldimmoniumchlorid | 1 | 2 | 3,5 | 4 | 5 |
| Cetylstearylalkohol | 1 | -- | -- | 0,5 | -- |
| Cetylalkohol | 1 | 2,5 | 2 | -- | 0,5 |
| Dimethylpolysiloxan | 1 | 1 | 1 | 3 | 5 |
| Nachtkerzenöl | 2 | -- | -- | 7,5 | -- |
| Tafelparaffin | 1 | 2,5 | 1 | -- | -- |
| Mineralöl, dünnflüssig | 2,5 | -- | 1 | 0,25 | 3,9 |
| Mineralöl, dickflüssig | 3 | 5 | 6 | -- | -- |
| Vaseline | -- | 4,5 | 4 | -- | -- |
| Methylisothiazolinone | -- | 0,2 | 0,2 | -- | -- |
| Dihydronepetalacton | 2,5 | 5 | 7,5 | 10,0 | 15,0 |
| Glycerin | 10 | 15 | 12 | 25 | 8 |
| Behenylalkohol | 1 | -- | -- | -- | 1 |
| Stearylalkohol | 1 | 1 | -- | -- | 1 |
| Methylparaben | 0,1 | -- | -- | 0,1 | 0,25 |
| Benzylalkohol | -- | 1 | -- | 0,5 | -- |
| Hydrierte Cocosfettglyceride | 2 | - | - | 1 | -- |
| Sheabutter | -- | 2 | -- | 1 | -- |
| Butylenglycol Dicaprylat/Dicaprat | 1 | - | - | - | 5 |
| Caprylic/Capric Triglycerid | --- | 4 | 3 | - | 1 |
| Ethylhexylkokosfettsäureester | 3 | -- | -- | 5 | -- |
| Octyldodecanol | -- | -- | 3 | -- | -- |
| Octamethyltetrasiloxan | -- | 1 | -- | 1 3 | -- |
| Hafermehl | -- | 1,5 | 1 | 1 | -- |

(fortgesetzt)

| Beispiel Nummer | 51 | 52 | 53 | 54 | 55 |
|---|---|---|---|---|---|
| EDTA | 0,5 | -- | -- | 1 | -- |
| BHT | 0,03 | -- | -- | 0,05 | -- |
| Dicarprylylcarbonat | -- | 5 | -- | -- | 2 |
| Parfüm | q.s. | q.s. | q.s. | q.s. | q.s. |
| Wasser | Ad 100 | Ad 100 | Ad 100 | Ad 100 | Ad 100 |

**Gele**

| | 56 | 57 | 58 | 59 | 60 |
|---|---|---|---|---|---|
| Sorbitan Stearate | 1 | 0,5 | 1 | 1,2 | 1,3 |
| Cetearylalkohol + PEG-40 Castor Oil + Natriumcetearylsulfat | 2,5 | 3 | 3,2 | 3,5 | 4 |
| Dihydronepetalacton | 5,0 | 11,0 | 7,5 | 5,0 | 20,0 |
| Phenaxyethanol | 0,3 | 0,8 | 0.4 | 0,6 | 0,4 |
| Methylparaben | 0,1 | 0,3 | 0,2 | 0,3 | 0,2 |
| Natriumlactat | 2 | 2 | 1,5 | -- | -- |
| Harnstoff | 5 | -- | -- | -- | -- |
| Glycerin | 3 | 7,5 | 5 | 7,5 | 5 |
| Serin + Glycine+ Alanin + Lecithin + Disodium Phosphat + Potassium Phosphat | 2 | 0,1 | 0,25 | 1 | 2 |
| Milchsäure | 2 | 2,2 | 1,1 | 0,5 | 1,1 |
| Xanthangum | 0,3 | 0,2 | 0,4 | 0,4 | 0,2 |
| Cetearylalkohol | 3 | -- | -- | -- | -- |
| Vaseline | 1 | 0,1 | -- | 1 | 5 |
| Mineralöl | -- | 10 | 10 | -- | -- |
| Parfum | q.s. | q.s. | q.s. | q.s. | q.s. |
| Ethanol | 10 | 3 | 4 | 5 | 10 |
| Wasser | ad 100 | ad 100 | ad 100 | ad 100 | ad 100 |

**Spray**

| | 11 | 12 | 13 | 14 | 15 |
|---|---|---|---|---|---|
| Dihydronepetalacton | 5 | 10 | 40 | 12,5 | 20 |
| Hammamelis-Extrakt | 0,2 | -- | -- | 1 | 0,2 |
| Aloe Vera Extrakt | -- | -- | 1 | 1 | 5,0 |
| Tocopherol | 0,5 | 0,5 | 0,75 | 0,25 | 0,1 |
| Meeresextrakt | 0,5 | -- | 0,75 | 3,0 | 0,25 |
| Panthenol | 0,5 | -- | 0,75 | 0,25 | 0,1 |

(fortgesetzt)

| | 11 | 12 | 13 | 14 | 15 |
|---|---|---|---|---|---|
| Carbomer | 0,05 | 0,35 | -- | -- | -- |
| Parfum | q,s, | q,s, | q,s. | q,s, | q,s, |
| Sorbitol | 10 | -- | -- | 5 | -- |
| Butylenglykol | -- | -- | -- | 5 | 10 |
| Propylenglykol | -- | -- | 10 | 5 | -- |
| Glycerin | -- | 7,5 | -- | -- | -- |
| Wasser | ad 100 | ad 100 | ad 100 | ad 100 | ad 100 |

## Patentansprüche

1. Zubereitung umfassend einen oder mehrere Insektenrepellentwirkstoffe gewählt aus Dihydronepetalactonen und/oder Extrakten der Katzenminze und ein oder mehrere Substanzen mit einem log P-Wert im Bereich von - 2,5 bis +2,5.

2. Zubereitung nach Anspruch 1, **dadurch gekennzeichnet, dass** der Anteil an Repellentien 5 Gew% und mehr umfasst, bezogen auf die Gesamtmasse der Zubereitung.

3. Zubereitung nach einem der vorstehenden Ansprüche, **dadurch gekennzeichnet, dass** die Zubereitung eine aufgereinigte Repellentienmischung, die zu etwa 90 Gew.% Dihydronepetalacton und 2 Gew.% Dehydronepetalacton umfasst, bezogen auf die Mischungsmasse, enthält.

4. Zubereitung nach einem der vorstehenden Ansprüche, **dadurch gekennzeichnet, dass** die Zubereitung ein oder mehrere weitere Insektenrepellentien enthält.

5. Zubereitung nach einem der vorstehenden Ansprüche, **dadurch gekennzeichnet, dass** als Substanzen mit einem log P-Wert von -2,5 bis +2,5 eine oder mehrere Verbindungen gewählt werden aus der Gruppe der mehrwertigen Alkohole, lineare Alkohole, verzweigte Alkohole, C5-C10 Alkylalkohole, Polyglycerine und/oder hydrophile Wirkstoffe.

6. Zubereitung nach einem der vorstehenden Ansprüche, **dadurch gekennzeichnet, dass** die Zubereitung ein oder mehrere Parfuminhaltsstoffe gewählt aus der Gruppe 1,3,4,6,7,8-Hexahydro-4,6,6,7,8,8-hexamethylcyclopenta-gamma-2-benzopyran, 2,6-Dimethyl-7-octon-2-ol, 2-Acetonapthone-1,2,3,4.5,6,7,8-Octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Famesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin umfasst.

7. Zubereitung nach einem der vorstehenden Ansprüche, **dadurch gekennzeichnet, dass** die Zubereitung ein oder mehrere Parfuminhaltsstoffe gewählt aus der Gruppe Hexylsalicylat. Linalylacetat und/oder 2-Isobutyl-4-hydroxy-4-methyltetrahydropyran umfasst.

8. Zubereitung nach einem der vorstehenden Ansprüche, **dadurch gekennzeichnet, dass** die Zubereitung maximal 0,3 Gew.% bakterizide-, fungizide-, sporicidally wirksame Mittel, Konservierungsmittel und/oder Formaldehyd und Formaldehyddonatoren enthält.

**9.** Zubereitung nach einem der vorstehenden Ansprüche, **dadurch gekennzeichnet, dass** die Zubereitung frei von bakteriziden-, fungiziden-, sporicidally wirksamen Mitteln, Konservierungsmitteln und/oder Formaldehyd und Formaldehyddonatoren ist.

**10.** Zubereitung nach einem der vorstehenden Ansprüche, **dadurch gekennzeichnet, dass** die Zubereitung frei von Parabenen ist.

**11.** Zubereitung nach einem der vorstehenden Ansprüche in Form eines Hydrodispersionsgel.

**12.** Zubereitung nach einem der vorstehenden Ansprüche in Form einer W/O- oder W/S-Emulsion.

**13.** Zubereitung nach einem der vorstehenden Ansprüche 1 bis 11 in Form einer O/W oder S/W-Emulsion.

**14.** Verwendung einer Zubereitung nach einem der vorstehenden Ansprüche als Insektenabwehrmittel.

**15.** Verwendung einer Zubereitung nach einem der Ansprüche 1 bis 13 als Insektenabwehrmittel in einer Verdunstungsvorrichtung.

**16.** Verwendung einer Zubereitung nach einem der vorstehenden Ansprüche 1 bis 13, **dadurch gekennzeichnet, dass** die Zubereitung als Tränkungsmedium auf Vlies, Pflaster oder Tuch aufgetragen wird.

Europäisches Patentamt
European Patent Office
Office européen des brevets

# EUROPÄISCHER RECHERCHENBERICHT

Nummer der Anmeldung
EP 08 00 7830

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| P,X | WO 2007/133683 A (DU PONT [US]; SCIALDONE MARK A [US]; PALEFSKY IRWIN [US]) 22. November 2007 (2007-11-22) * Seite 57 - Seite 58; Ansprüche; Beispiele * | 1,2,4,5, 8-10, 12-14,16 | INV. A01N43/16 A01N65/00 A01P17/00 |
| X | WO 2006/050519 A (DU PONT [US]; SCIALDONE MARK [US]) 11. Mai 2006 (2006-05-11) * das ganze Dokument * | 1-16 | |
| D,X | WO 2005/034626 A (DU PONT [US]; HALLAHAN DAVID L [US]; MANZER LEO E [US]) 21. April 2005 (2005-04-21) * das ganze Dokument * | 1-16 | |
| X | EP 1 738 744 A (BEIERSDORF AG [DE]) 3. Januar 2007 (2007-01-03) * das ganze Dokument * | 1-16 | |

RECHERCHIERTE SACHGEBIETE (IPC)
A01N

Der vorliegende Recherchenbericht wurde für alle Patentansprüche erstellt

1

| Recherchenort | Abschlußdatum der Recherche | Prüfer |
|---|---|---|
| München | 15. Januar 2009 | Bertrand, Franck |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 00 7830

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-01-2009

| Im Recherchenbericht angeführtes Patentdokument | Datum der Veröffentlichung | Mitglied(er) der Patentfamilie | Datum der Veröffentlichung |
|---|---|---|---|
| WO 2007133683 A | 22-11-2007 | US 2007264297 A1 | 15-11-2007 |
| WO 2006050519 A | 11-05-2006 | AU 2005301975 A1 | 11-05-2006 |
| | | BR PI0516881 A | 23-09-2008 |
| | | CA 2584354 A1 | 11-05-2006 |
| | | CN 101090630 A | 19-12-2007 |
| | | EP 1806969 A1 | 18-07-2007 |
| | | JP 2008519050 T | 05-06-2008 |
| | | KR 20070083916 A | 24-08-2007 |
| WO 2005034626 A | 21-04-2005 | AU 2003270748 A1 | 27-04-2005 |
| | | BR PI0318501 A | 12-09-2006 |
| | | CA 2538363 A1 | 21-04-2005 |
| | | CN 1838881 A | 27-09-2006 |
| | | EP 1681925 A1 | 26-07-2006 |
| | | JP 2007521240 T | 02-08-2007 |
| EP 1738744 A | 03-01-2007 | DE 102005030016 A1 | 11-01-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2246433
- DE 19645250
- DE 19645920
- WO 2006096876 A
- WO 2005034626 A
- DE 102005033845


### In der Beschreibung aufgeführte Nicht-Patentliteratur


- **SEYDEL ; SCHAPER.** Chemische Struktur und biologische Aktivität von Wirkstoffen. Verlag, 1979
- **PURCELL ; BASS ; CLAYTON.** Strategy of Drug Design: A guide to biological Activity. J. Wiley and Sons, 1973
- **REKKER ; MANNHOLD.** Calculation of Drug Lipophilicity. Verlag, 1992
- **KUBINY, H.** QSAR: Hansch Analyses and Related Approaches. vol. 1, 5. 77 ff
- **BREHM, O. ; OHLMEYER, I. ; FELS, G.** Automatisierte pKa- und logP-Bestimmung. *GIT Labor-Fachzeitschrift,* 1997, vol. 41, 368-374